# EUROPEAN PATENT SPECIFICATION

(11) **EP 1 351 613 B1**
(45) Date of publication and mention of the grant of the patent: **08.03.2006**
(21) Application number: 01995518.6
(22) Date of filing: 30.11.2001
(51) Int. Cl.: A61B 17/64

(54) **EXTERNAL SKELETAL FIXATOR**
EXTERNER FIXATEUR
SYSTEME DE FIXATION DU SQUELETTE EXTERNE

(30) Priority: 04.01.2001 YU 801
(43) Date of publication of application: 15.10.2003
(73) Proprietor: Mitkovic, Milorad, 18000 Nis (YU)
(72) Inventor: Mitkovic, Milorad, 18000 Nis (YU)
(74) Representative: Matschnig, Franz
(86) International application number: PCT/YU2001/000030
(87) International publication number: WO 2002/053038

(56) References cited:
- US-A- 2 393 694
- US-A- 4 483 334
- US-A- 4 848 368

## Description

### TECHNICAL FIELD Of THE INVENTION

The invention relates to an external skeletal fixator, which comprises at least two pins (4), that can be placed in each of the main bone fragments.

It is already well known fact that the external fixation makes possible the fixation of bones by the performed using of special pins, or wires, screwed or introduced into the bone fragments out of the fracture area, and thus avoiding the penetration of the foreign materials into the fracture site.

There are various types of external fixators. Most of them do not provide closed fracture reduction; only few types of existing external fixators provide possibility of closed fracture reduction or postoperative correction of the reduction. Among these latest devices are the following: "Orthofix", Hoffmann's, Scherer's, IIizarov's and the others.

One of the most famous external fixator is Hoffmann's. It consists of at least two clamps used for the fixation of the pins screwed into the bone, longitudinal bar and a ball-like joint that establishes the connection between the clamp and the bar. The clamp may be used for the fixation of two to five pins. While being screwed into the bone, the pins have to be parallel and at the exactly designated mutual distance that is determined in advance, and that depends on the gutters on the clamps. The bar may be divided in two parts so that - by the addition of some special nuts and screws - it may be shortened or made longer. The elements of this system may be combined and thus build the constructions of various complexities.

However, Hoffmann's system for the external fixation of bones has, as all above mentioned systems, three main disadvantages, as follow:
~ the conventional pins have to be parallel, and placed on the predetermined mutual distance, so that, in order for them to be applied, a special guidance has to be used.
~ this system doesn't provide possibility of accurate closed fracture reduction (without opening of the fracture area).
~ the frame is unilateral and pins are in one plane which provides non balanced stability so that the fixed bone is very stable in the plane of the pins and several times (5-20 times) less stable in the perpendicular plane. Provided stability of the fractured bone stabilized by the use of this type of external fixator is not natural and not optimal because long bones as tibia or femur has the same stability during the action of the forces in antero-posterior or lateral direction.

American patent No. US 2,393,694 (Kirschner 1946) discloses an external skeletal fixator comprising clamps for carrying pins, which clamps are mounted on carriers. The clamps can slide along the and rotate around the respective carrier. Each carrier carries two clamps.

American patent No. US 4,848,368 (Kronner 1989) describes a universal external fixation frame including of rod-holder bodies, a pin holder body for each rod-holder body connected to the rod holder body through a universal swivel mechanism.

There is an apparatus that enables the well balanced three-dimensional stability, presented in Yugoslavian patent No YU 48736 whose inventor is Mitkovic Milorad, who is the author of this invention as well. The main disadvantage of the apparatus, according to the patent YU 48736, lies in the fact that between the carrier of the clamp and the bar exists the effect of the self-locking, which disturbs the function of the fixator during performing of compression or distraction or during the so called "dynamization" process. The disadvantages of the patent YU 48736 are also lack of the compression-distraction possibilities and adding of an additional carrier of the clamp to the bar after already set apparatus to the patient. This disadvantage has been solved by the construction of new carrier of the clamp with open hole for the bar on the head of the carrier. Disadvantages regarding compression-distraction have been solved by construction of compression-distraction bar and by another solution - removable compression-distraction device.

The object of the invention is to provide an external, skeletal fixator which renders it possible to use conventional pins without any kind of guidance, whereby the pins will have the three-dimensional freedom of movement, while the whole construction provides a balanced three-dimensional stability during which the apparatus is highly adjustable, suitable for the compression and distraction and manageable for clinical application and production.

This object is achieved by means of an skeletal fixator according to claim 1.

The three above mentioned primary disadvantages of the external fixator with the parallel pins, and the external fixator according to the patent YU48736 are eliminated by means of the new construction of the external skeletal fixator according to the invention. At the same time a simplification of the application and production of the elements for the external fixation is achieved.

Further advantageous embodiments of the invention are described in the dependent claims 2-10.

### DETAILED DESCRIPTION OF THE INVENTION

The primary goal of the invention is finding the solution to the problem of the balanced three-dimensional fixation of the fractures, dynamisation, accurate closed fractures reduction, high mobility and independent introduction of any pin. Apart from that, the application and managing of the fixator has to be simple, even for a beginner, as well as easy for the production.

The essence of the invention lies in the achievement of the free and reliable sliding of the carrier of the clamp in relation to the bar, securing the conditions for the compression and distraction, whereby, there is neither the phenomenon of self-locking nor the looseness among the components. This is accomplished by the construction of the carrier of the clamp that is put on the bar over the cylindrical hole, so that the sliding of the carrier of the clamp across the bar is going on smoothly and without self-locking; whereby, there is no excessive looseness between the bar and the carrier of the clamp, disregarding the fact whether the screw, used for the fixation of the carrier of the clamp to the bar, is more or less unscrewed, or is entirely unscrewed and separated from the carrier of the clamp. This enables even the possibility of compression and distraction across the carrier of the clamp. Likewise, the essence lies in the achievement of the three-dimensional freedom of the application of each pin. The frame of the fixator may always be placed, regardless of the position of already applied pins through the bone. This is made possible by the construction of the clamp, which has rotator and sliding freedom in relation to the pin and to the carrier of the clamp and rotator and sliding freedom of the carrier of the clamp in relation to the bar, whereby, clamp can be fixed to the pin and to the carrier of the clamp and carrier of the clamp can be fixed to the bar. After the application of at least two pins to each main fragment of the bone, disregarding the direction of their application, it is possible to set a frame of the fixator that consists of the bar on which the assembly: carriers of the clamps and clamps are placed. The author of both this method and this invention recommends that the pins in the tibia to be applied convergently, mutually closing the angle of 90°. Afterwards, the precise setting of the position of the fragments is carried out by the sliding movement of the carrier of the clamp across the bar. When the desired position of the bone fragment is achieved, all screws and nuts are strongly tightened. Later on, the distraction or compression may be carried out by the special additional device, whether by using the telescopic or ordinary bar.

According to this invention, the external skeletal fixator has many advantages, among which the most important are the following:
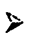 during the application of the pins, there is no need for any kind of guidance
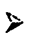 the frame can always be attached to the pins, disregarding their position,
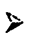 it is highly adjustable and it can be used as accurate reduction device for the precise fixation of the fracture,
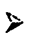 the later dynamisation of the fixator is possible as well,
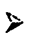 it ensures three dimensional balanced biomechanical conditions for the faster healing of the fracture,
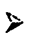 it enables the lengthening of the bones, the correction of the deformities and dynamisation,
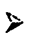 easy and simple handling.

### BRIEF DESCRIPTION OF THE DRAWINGS

In order to understand the invention easily, and to show how it can be realized in practice, the author refers to the project drawings attached herewith and related to the internal bone fixation device, wherein:
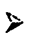 *Figure 1* presents assembly of invention, observed in axonometric view,
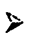 *Figure 2* expansively presents elements of carrier of clamp, observed in axonometric view,
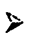 *Figure 3* shows axonometric view of screw from figure 2
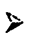 *Figure 4* shows pressing element from figure 2
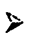 *Figure 5* presents profile look of pressing element from figure 4 viewed in vertical projection
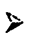 *Figure 6* shows composite in vertical projection of carrier of clamp from figure 2 with bar
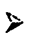 *Figure 7* shows vertical cross-section of carrier of clamp carried out along line VII - VII from figure 6
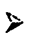 *Figure8* shows cross-section of carrier of clamp carried out along line VIII - VIII from figure 7
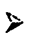 *Figure 9* shows cross-section of carrier of clamp carried out along line IX - IX from figure 7
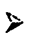 *Figure 10* shows screw and pressing element according to second performance, observed in axonometric view,
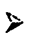 *Figure 11* shows horizontal projection of carrier of clamp according to second performance, with horizontal cylindrical supporting head of tubular carrier
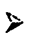 *Figure 12* shows horizontal projection of carrier of clamp according to third performance, with ball-like supporting head of tubular carrier
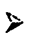 *Figure 13* shows horizontal projection of complex of longer screw coupled with pressing element, designed to be coupled furr with tubular carrier from figure 11 and 12
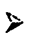 *Figure 14* shows anor way of performance of carrier of clamp with paralelopipedic supporting head and bar-like carrier and threaded extension. shown in axonometric view
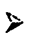 *Figure 15* shows clamp for pin in expansion view, viewed axonometrically
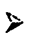 *Figure 16* axonometrically shows telescopic compressively - distractional device
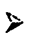 *Figure 17* expansionally shows plate-like compressively-distractive device according to second variation
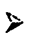 *Figure 18* expansionally shows clamp for fixation of two bars in various directions viewed axonometrically
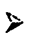 *Figure 19* shows schematic presentation of complex of bars and clamp from figure 18 viewed axonomerically

### DETAILED DESCRIPTION OF THE INVENTION

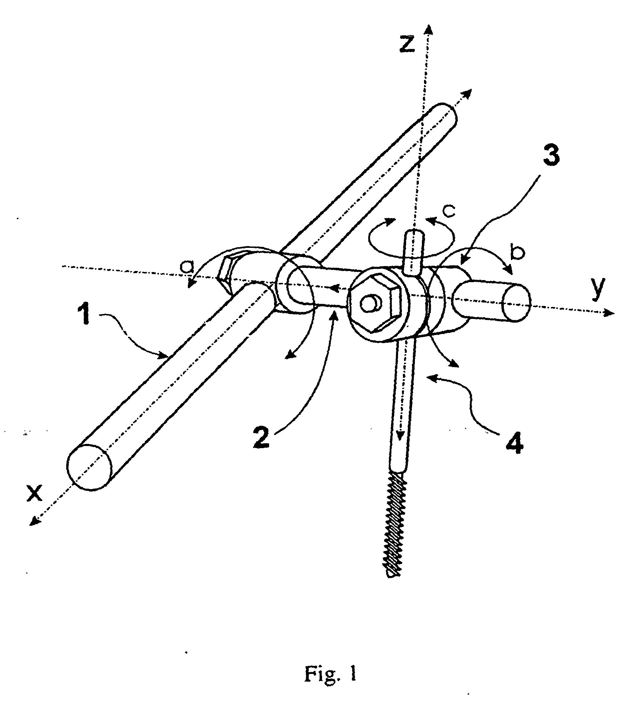

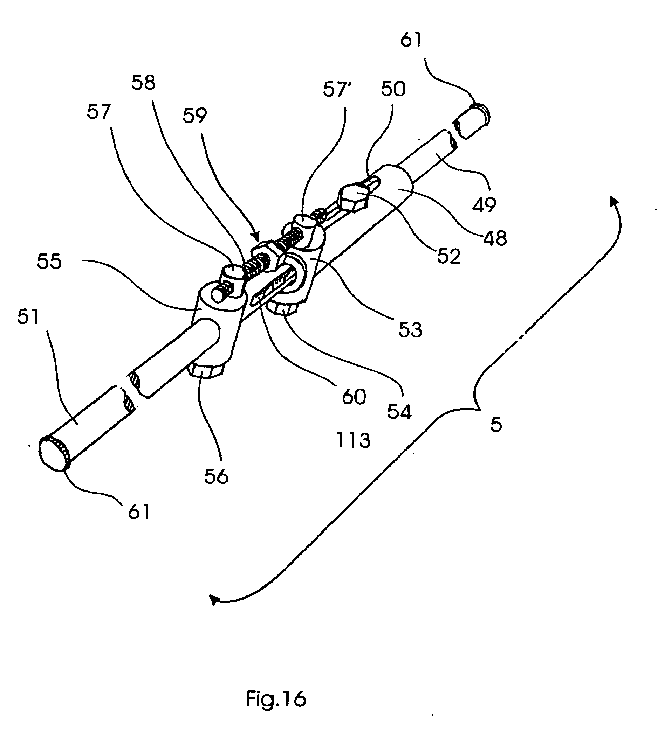

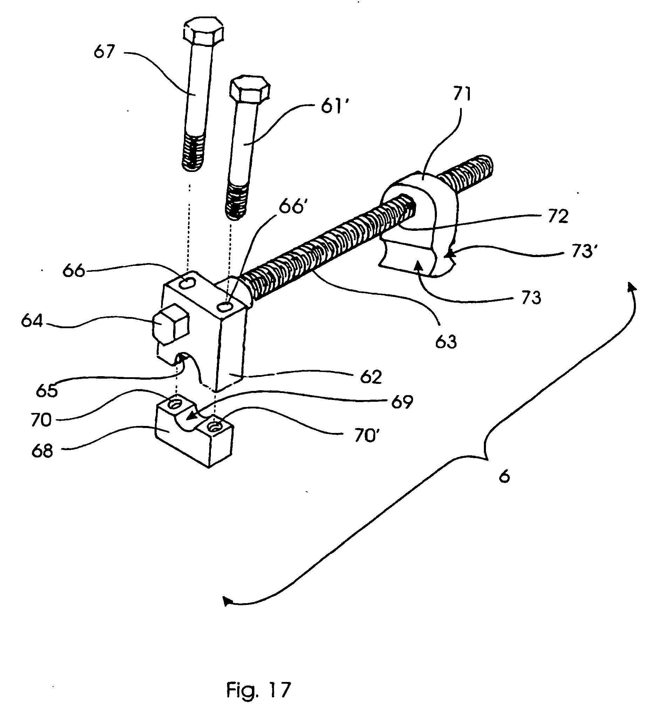

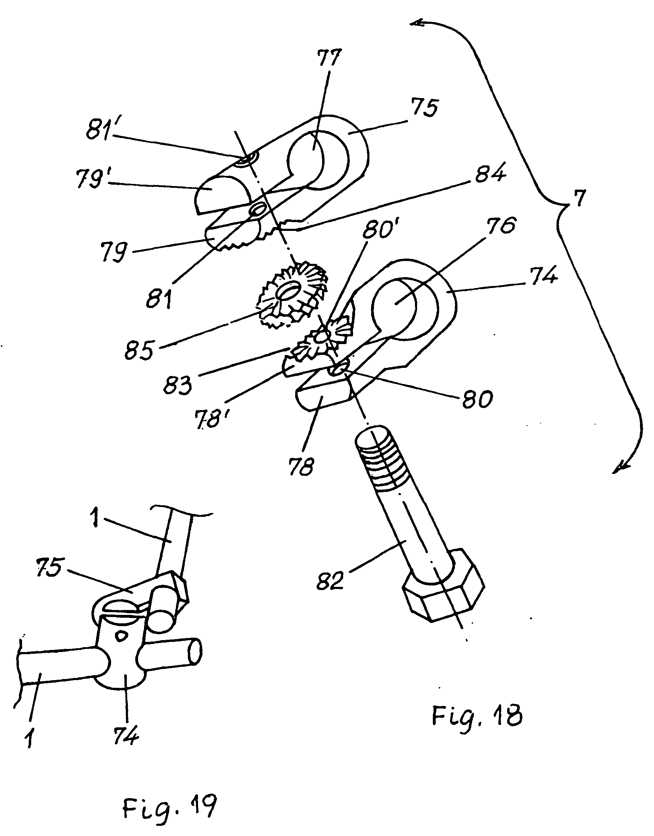

The observation of figure 1 of the enclosed drawing may be viewed by perceiving external skeletal fixator that consists of bar 1, carrier of clamp 2 of clamp 3 and pin 4. The extended complex of fixator includes telescopic and plate-like comressively-distractional device 5, 6, shown by figures 16 and 17, and special clamp 7 for simultaneous tightening of two bars 1, shown by figures 18 and 19.

The bar of external skeletal fixator is circular cross-sectioned bar on which carrier of the clamp 2 is pulled; before fixation, carrier of clamp can be moved in both directions along bar 1, or it can be moved rotationally around bar 1. On carrier 2 of clamp, adjustable clamp 3 is pulled on; it is at same time axially and rotationally movable, both in relation to carrier of clamp 2 and in relation to pin 4, which is screwed into bone.

The translation movements of elements of external skeletal fixator is shown by figure 1 of drawing designated by arrows marked as X, Y and Z, while rotational movements are marked by a, b and c.

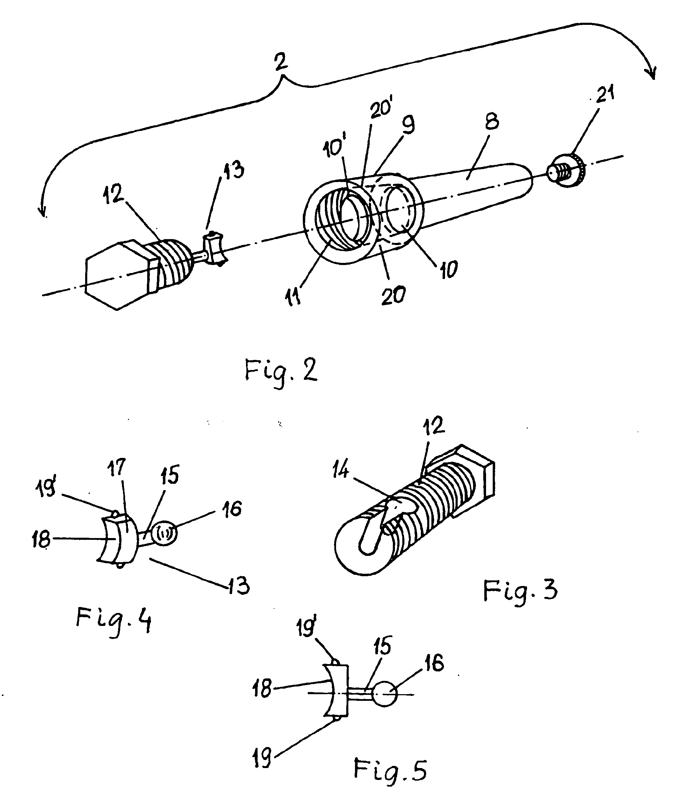

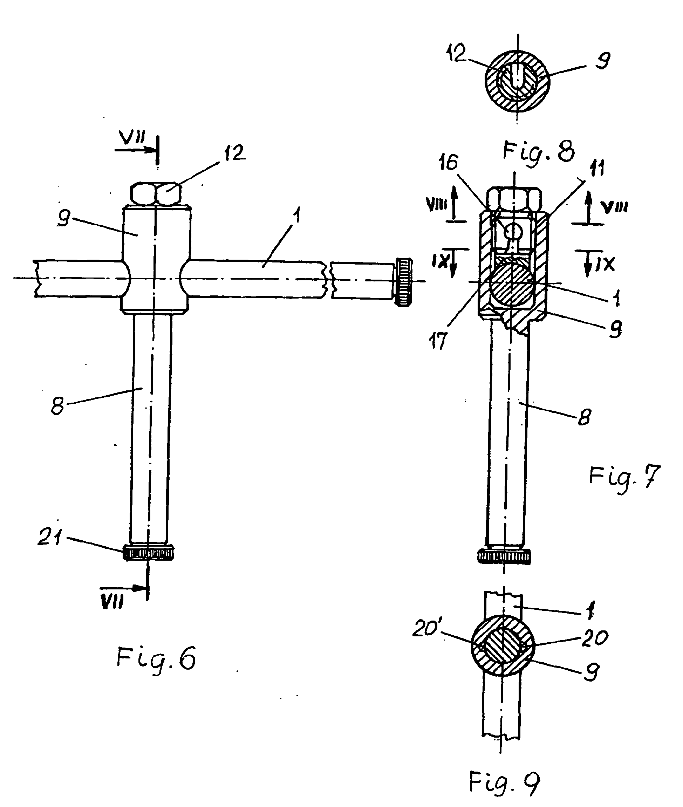

The new construction of carrier 2 of clamp, shown by figures from 2 to 9, includes circular bar-like body 8, which, on one of its ends, has coaxially widened cylindrical extension 9, through which circular holes 10 and 10' are vertically pierced. The middle axes of bar-like body 8 and circular holes 10, 10' are mutually perpendicular. The body 8 is used for carrying of clamps 3, whereas holes 10 and 10' for connection to bar 1. Inside cylindrical carrying head 9, thread 11 is made, in which screw 12 is screwed with profile-like pressing element 13 situated inside gutter 14 on screw 12. The shape of gutter 14 reminds of keyhole. The pressing element 13, separately shown by figures 4, 5 and 7, consists of cylindrical neck 15 that on one of its ends has ball 16, while on other end cylinder 17 with pressing surface 18 which is negative - cylinder shape. The ball 16 and larger part of neck 15 are placed inside gutter 14, while negative-cylinder 17, which serves purpose of better fixation of bar I to carrier 2 of clamp, occupies forward position in relation to gutter 14.

By screwing screw 12, pressing element 13 is pushed until negative-cylinder 17 is not placed directly onto bar I introduced through circular holes 10 and 10' on extension 9 of carrier 2 of clamp. It goes without saying that diameters of negative-cylinder 17 and bar 1 have to be identical. While unscrewing screw 12, cylinder 17 of pressing element withdraws as well, so that connection between carrier 2 and bar I is unlocked. This new constructional solution eliminates phenomenon of self-locking, which contributes to new characteristic of fixator itself. In order for prevention of twisting of negative-cylinder 17, during screwing of screw 12, and for purpose of making concave surface 18 of negative-cylinder 17 always lie properly on bar 1, cylinder 17 is led across two symmetrical wartlike convexes 19 and 19' that go inside appropriate side gutters 20 an 20' led out inside extension 9 of clamp 2.

On opposite end of bar-like body 8, a smaller screw 21 is screwed with lens-like head whose diameter is larger than one of body 8. This occurs in order for clamp 3 not to slide down body 8.

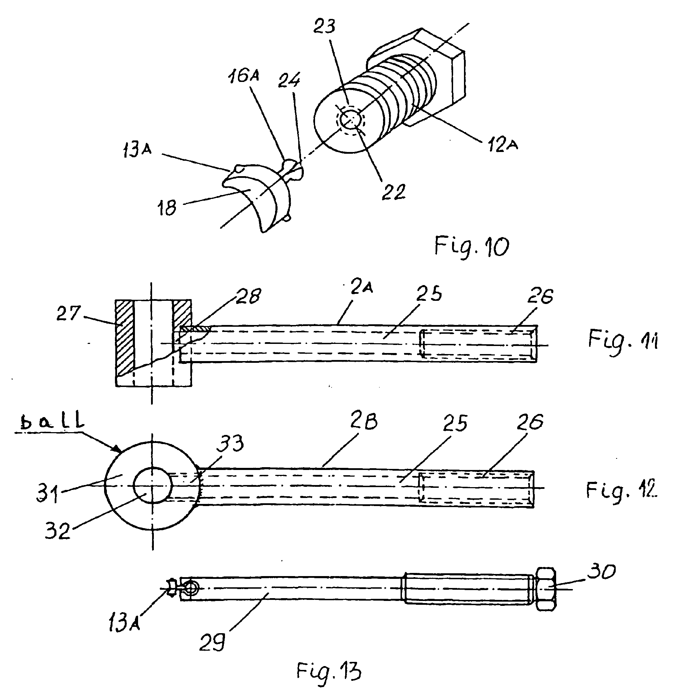

Figure 10 of drawings shows other variation of threaded screw 12A and negative-cylindrical pressing element 13A. According to this variant, screw 12A has no profile gutter 14; yet, it has smaller hole 22 pierced beginning from end of trunk, on which a spherical expansion 23 is spherically continued. The negative-cylindrical pressing element 3A is altered as long as his ball 16A is vertically cut forming slit 24. The connection of both elements is achieved by placing cut ball 16A through narrower hole 22 as well as its placing inside spherical expansion 23, out of which it cannot break loose easily, once it penetrates there.

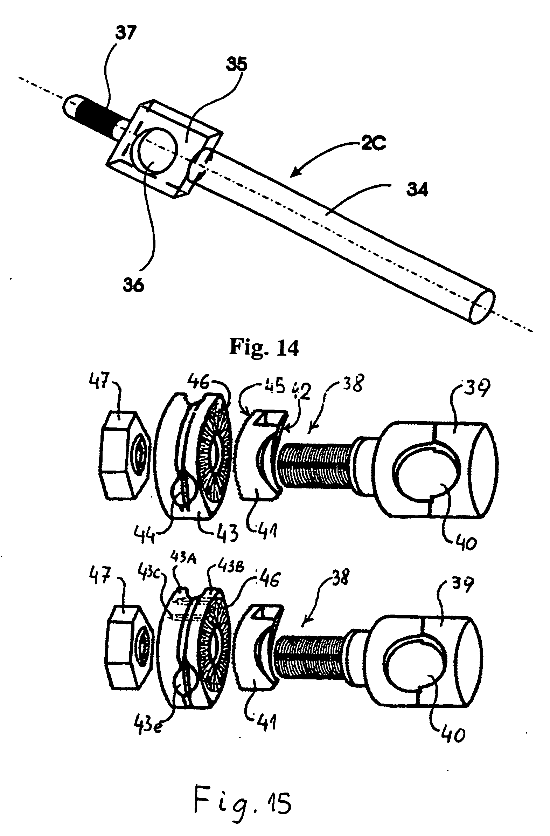

The second variation of carrier 2A of clamp (see figure 11) consists of bar-like circular tubular body 25 with inner thread 26 led out on one of its parts beginning from free end. On other end of body 25, cylindrical supporting head 27 is fixed, whose inner diameter is considerably bigger than diameter of bar 1. The long axis of symmetry of tubular body 25 and cylindrical supporting head 27 are mutually perpendicular, whereby, on their parts through supporting head 27, circular hole 28 is led out. Through tubular body 25, screwed vertical screw 29 is screwed which, on one of its ends has inserted pressing element 13A which penetrates to hole 28. The hexagonal head 30 of already mentioned long screw 29, prevents slipping of clamp 3 out of carrier 2A of clamp.

The third variation of carrier 2B of clamp (see figure 12) also consists of circular tubular body 25 with inner thread 26, with only difference that supporting head 31 has ball-like shape with cylindrical hole 32 for bar 1. The long axles of symmetry of body 25 and cylindrical hole 32 are mutually perpendicular. Through tubular body 25, screw 29 with pressing element 13a, that penetrates to hole 33 on ball 31, is introduced, whereby axises of hole 33 and tubular body 25 are in coaxial relation.

The fourth variation of carrier 2C includes circular bar-like body 34 on which is parallelopipedic supporting head 35 with horizontal hole on bar 1. On other end of supporting head 35 is extension with shorter threaded bar 37 which serves for admission of clenching components which is pressed by corresponding nut producmg locking of carrier 2C of clamp to bar 1. The coaxial axis of body 34, supporting head 35 and threaded bar 37 are all perpendicular to vertical axis of cylindrical hole 36 on supporting head 35 (see figure 14). This carrier of clamp is suitable for AO external fixator.

The clamp 3 of external skeletal fixator, shown by figure 15 of drawing, consists of special screw 38 with cylindrical head cut on both sides, through which cylindrical hole 40 is horizontally pierced; it is used for introduction of bar 1 or carrier 2, 2A or 2B. On cut part of cylindrical head 39, a rider 41 is pulled on, which, with its negative bar-like surface 42, penetrates cylindrical hole 40. Behind rider 41, on screw 38, two circularly-ringed plates 43 which form channel 44 for pin 4. It can be constructionally achieved that plates 43 be separated or conjugated. The lying surfaces of rider 41 and plates 43 have tooth-like surfaces 45 and 46; by fastening of screw 47, their tooth-like surfaces eliminate possibility of rotation of plate 43 in relation to rider 41.

The application technique of external skeletal fixator according to this invention is very simple. After ordinary preparation of operating field and cleaning of eventual wound, approximate reduction of fracture is performed, by open or closed method. For example, first thing that may be done is making four incisions (two on each fragment of fractured bone), then, by using drill, four holes are made, in which pins 4 are fixed or pins are introduced directly without praedrilling. The position of all pins 4 is optional. However author recommends o that pins are convergent forming angle of 45° to 90°.

The preparation of external skeletal fixator for above cited example consists of pulling of four carrier 2 of clamp, with clamps 3 pulled on them as well, on bar 1. The fixator prepared in this way, as is shown by figure 1, is brought above pins 4, since each clamp 3 is adjustably pulled on each pin 4, across gutter 44. After adjustment of position of clamps 3 on pins 4, and carrier 2 on bar 1 are performed, fixation of components of fixator is carried out by screwing of nuts 47 and screws 12. If further correction of position of bone fragments is required, it can be carried out several times. It is enough to loose both clamps 3 that support one of bone fragments and loose carriers 2 from bar 1. The removal and placement of fixator during operation or postoperatively is as well possible and relatively simple.

In cases where it is necessary for two bone fragments to be closer to each other, or to be separated (for example, during lengthening of bone), telescopic compression-distractional device 5 is used, according to this invention, which is shown by figure 16 of enclosed draft. The complex of device 5 consists of cylinder 48 which, on one of its ends, is open, and on other, it has coaxially fixed bar 49. On upper part of cylinder 48, longer gutter 50 is pierced. The long bar 51 is pulled through inner part of cylinder 48. The bar 49, on its far end has pierced thread hole (it is not visible in picture) in which, through gutter 50, tightening screw 52 is screwed into bar 51. It is important to emphasize that bars 49 and 51 of circular horizontal cross-section are coaxial and that they have same diameter which is identical to one of bar 1 of fixator. On cylinder 48, slider 53 is pulled on, which is fixed to cylinder 48 by screwing screw 54, while on bar 51, slide 55 is pulled on, tightened by screwing screw 56. On slider 53, there is extension 57', while on slider 55, identical extension 57; whereby, through each extension 57 and 57', horizontal thread hole is pierced (they are not marked in picture). In threaded holes on extensions 57 and 57', threaded spindle 58 is threaded with left and right thread, whereby, hexagonal head 59 for adjustment of distance between slider 53 and 55 is rambled or approached symmetrically in relation to middle of spindle. It is constructionally achieved that threaded spindle 58 is parallel to bars 49 and 51. The size of distance adjustment between sliders 53 and 55 can be read on scale 60 engraved on bar 51. The application of telescopic compression-distraction device 5 is very simple. On bars 49 and 51, at least two carriers 2 with clamps 3 on them are fixed, or at least two clamps 3 which, in their gutters 44, firmly surround pins 4 screwed into bone fragments. If it is necessary for bone fragments to come closer, than it is also necessary to unscrew tightening screw 52, which makes it possible for bar 51 to unlock. By screwing middle screw 59 on threaded spindle 58, what is achieved is coming together of slider 53 and 55 and telescopic penetration of bar 51 in cylinder 48, which may be controlled on scale 60. When size of penetration of bar 51 is achieved, it is necessary to screw tightening screw 52 and fix bar 51 to cylinder 48.

When it is necessary to separate bone fragments, sequence of techniques is identical, with difference that middle thread 59 has to be unscrewed apropos rotated in opposite direction in relation to thread on spindle 58. Then, bar 51 goes out from cylinder 48, or sliders 53 and 55 are separated, which brings about separation of their bars 49 and 51. Since clamps 3 are fixed to bars 49 and 51, separation between pins 4 is achieved, which has been goal of device 5. In order to prevent slipping out of carrier 2 and clamp 3 from bars 49 and 51, small screws 61 with lens-like head are screwed on end of bars 49 and 51.

The second variation of compression-distraction device 6 is based on plate-like elements coupled with threaded spindle and shown by figure 17 of enclosed drawing.

The complex of device 6 consists of upper plate 62 with rotationally lying threaded spindle 63 whose rotation is performed by hexagonal head 64 leaned externally on plate 62. The upper plate 62 has on its top half-cylindrical gutter 65 and two vertically separated cylindrical holes 66, 66', designed for passage of screws 67 and 67' for connection with plate 68. The lower plate 68 has half-cylindrical gutter (identical to gutter 65) and two vertically separated threaded holes 70 and 70' that serve for connecting of both plates 62, 68, by screws 67, 67'. The upper and lower plate 68 and 69 are placed around bar 1 of fixator so that they are flanked by their gutters 65 and 69. By tightening of screws 67 and 67' firm connection is achieved between bar 1 and plates 62 and 68. In complex of device, there is another profile plate 71 with threaded hole 72, whereby plate 71 is screwed on screw 63. In lower part of plate, concave side gutters 73, 73' are led out on both sides, and they lie along cylindrical supporting head 8 of carrier 2 of clamp. The rotating of head 64 of screw 63 enables approaching or separation of plates 71, or pushing away or coming closer of carriers 2 on which clamp 3 and pins 4 are fixed, depending on direction of rotation of head 64.

In end, figure 10 of drawing shows special double joint 7 for fixation of two bars 1 in various positions. The double joint has two joining parts 74 and 75 with cylindrically cut holes 76 and 77 through which two bars 1 can be introduced (see figure 19). The legs 78, 78' which are connected to hole 76, and legs 79, 79' of hole 77 have coaxial holes 80, 80' and 81, 81' that serve for screwing of screw 82. On leg 78', around hole 80', on joining part 74, radial teething 83 is carried out; on leg 79, around hole 81 of joining part 75, similar teething has been carried out. Between connecting parts 74 and 75, that is, between teething 83 and 84, mutually tooth-like circularly-ringed plate 85 is placed. The hole 81' on leg 79' of joining part 75 is threaded so that screw 82 is screwed into threaded hole 81', which produces tightening of all elements of double joint 7. Since tooth-like areas 83 and 84 are in accordance with tooth-like areas on tile 85, by rotating of joining parts 74 and 75, various positions of bar 1 may be achieved and bars locked.

The special double joint 7 is especially applicable in joining of two fixators; for instance, one used for fixation of lower leg fracture with other used for fixation of upper leg, which prevents all possible movements inside knee.

### INDUSTRIAL OR OTHER APPLICATIONS OF INVENTION

From the description above and the enclosed drawings, the application of the invention is obvious, and there is no need for it to be particularly described. Everything that is previously quoted is at the same time tested and examined on prototypes of invention.

## Claims

1. An external skeletal fixator, which comprises at least two pins (4), that can be placed in each of the main bone fragments, the skeletal fixator further comprising for each pin (4) an own carrier (2) supporting a clamp (3), which clamp (3) is pulled overt one of the pins (4), wherein the clamp (3) is mounted on the carrier (2) in a manner that allows, for the purpose of adjusting the skeletal fixator, a sliding movement of the clamp (3) along the carrier (2) and a rotational movement of the clamp (3) around the carrier (2), and wherein the carrier (2) is mounted to a connective bar (1) in a manner that allows, for the purpose of adjusting the skeletal fixator, a sliding movement of the carrier (2) along the connective bar (1) and a rotational movement of the carrier (2) around the connective bar (1), wherein the connective bar (1) connects at least two carriers (2).

2. An external skeletal fixator according to claim 1, **characterized in that**, that the carrier (2) comprises a circular bar-like body (8) with a coaxially integrally continued cylindrical supporting head (9), which supporting head (9) comprises bilateral holes (10, 10') as a mounting device for the connective bar (1), wherein a screw (12) is screwed into the supporting head (9), which screw (12) comprises a pressing element (13) placed inside a profiled gutter (14) of a keyhole shape on a trunk of the screw (12), wherein the pressing element (13) comprises a cylindrical neck (15) with a ball (16) on one of its ends and a concave surface on the other end, whereby a radius of the concave surface (18) and a radius of the connective bar (1) are identical, wherein two notches (19, 19') arranged on opposite surface areas of the pressing element (13) for carrying vertical gutters (20, 20') are placed inside the cylindrical supporting head (9), and wherein a small screw (21) with a lens-like head is screwed on to the free end of the bar-like body (8), whereby the diameter of the lens-like head is bigger then the diameter of the bar-like body (8).

3. An external skeletal fixator, according to claim 2, **characterized in that**, the screw (12A) of the carrier (2) comprises a pressing element with a concave surface and a ball (16A), which ball (16A) has a split (24), wherein the ball (16A), squeezing through a narrower hole (22) is placed inside a spherical hole (23) extending coaxially with a trunk (12A) of the screw (12A).

4. An external skeletal fixator according to one of the claims 1 to 3, **characterized in that,** the carrier (2A) comprises a circular tube (25) on one end and a perpendicular cylindrical supporting head (27) on its other end, with a hole (28), for a screw (29) with a concave surface (13A) pulled through a circular hole (28), wherein the screw (29) has a rectangular head (30).

5. An external skeletal fixator according to claim 4, **characterized in that**, that the carrier (2B) has a ball-like supporting head (31) at one of its ends, with a transversely pierced cylindrical hole (32) for the connective bar (1), whereby the longitudinal axis of the tube (25) and the cylindrical hole (32) are mutually perpendicular, wherein a long screw (29) with a pressing element with the shape of a negative-cylinder (13A) is pulled and threaded through the tube (25) penetrating the cylindrical hole (33).

6. External skeletal fixator according to one of the claims 1 or 2 **characterized in that**, that the carrier (2C) comprises a circular bar (34), on which bar (34) a parallelopipedic supporting head (35) with a transversally pierced cylindrical hole (36) for the connective bar (1) is arranged, wherein shorter threaded bar (37) is arranged on the opposite end of the supporting head (35), wherein the shorter threaded bar is arranged coaxially with the axis of the carrier (34), wherein the axis of the threaded bar (37) and the bar (34) are mutually perpendicular to the axis of the cylindrical hole (36) of the supporting head (35).

7. An external skeletal fixator according to the claim 1, **characterized in that**, that the clamp (3) comprises a special screw (38) with a bilaterally notched cylindrical head (39), which head (39) comprises a cylindrical hole (40) for pulling on the body (8) of the carrier (2) or over the connective bar (1), wherein a rider (41) which goes into a cylindrical hole (40) with its negative-cylindrical surface (42) is slipped over the notched end of the cylindrical head (39) of the screw, that behind rider (41), two partly connected circularly ring-like plates (43) are pulled on screw (38), both of which have formed gutter (44) designed for its pulling on pin (4), whereby plates (43) may be separated and connected loosely, and that lying plate of raider (41) and plate (43) with teethed surfaces (45, 46) that are tightened by nut (47) on threaded trunk of screw (38).

8. An external skeletal fixator according to claim 1, **characterized in that**, it comprises a bar (5) comrising a cylinder (48) and a coaxially fixed external bar (49), whereby a gutter (50) is led out on the cylinder (48) from above, wherein the cylinder (48) is a partly, telescopically inserted into a connective bar (51) which is fastened by tightening screw (52) throughout the gutter (50), whereby the connective bars (49, 51) are of the same diameters as the connective bar (1), wherein a slider (53), is fastened by a screw (54) and pulled on the cylinder (48), whereby a further slider (55) fastened by a screw (56) is mounted on the external bar (51), wherein extensions (57, 57') of the sliders (53, 55) are fixed, by means of a threaded spindle (58) whereby a head of a screw (59) is fastened in the very middle of the threaded spindle (58), and wherein a scale is engraved between the sliders (53, 55) on the connective bar (51), and that on the connective bar (49), a pair of carriers (2) is pulled with the clamps (3) connected to the pins (4) of one bone fragment, whereas an other pair of carriers (2) with its clamps fastened to the pins (4) of an other main bone fragment.

9. An external skeletal fixator according to claim 8, **characterized in that**, it contains a plate-like device (6) consisting of an upper plait (62) with a rotationally lying threaded spindle (63) with a hexagonal head (64), and a lower plate (68), that comprises semi-cylindrical holes (65, 69) for enveloping the connective bar (1) are led out on the plates (62, 68), whereby the plates (62, 68) are firmly tightened by screws (67, 67') through cylindrical holes (66, 66') on an upper plate (62) and through threaded holes (70, 70') on the lower plate (68), wherein a threaded spindle (63) is screwed in a plate (71), through a thread hole (72), whose transversely concave gutters (73, 73') are reciprocally and are used for leaning of the head (9) of the carrier (2).

10. An external skeletal fixator according to claim 1, **characterized in that**, it comprises a double clamp (7) with two clamps (74, 75) with cylindrical holes (76, 77) for connective bars (1), whereby holes (76, 77) are cut in one place and are continued to two legs (78, 78') of a clamp (74) and the legs (79,79') of the other clamp (75), wherein circular holes (80, 80') are coaxially pierced through both legs (78, 78') and other circular holes (81, 81') are pierced through other legs (79, 79'), whereby two circular holes (81', 84) of the legs (78', 79) lying opposite to each other each comprise radially arranged teeth (83, 84), wherein a plate (85) comprising teeth on both of its sides is inserted between the two circular holes (81', 84), and wherein a screw (82) for tightening the clamps (74, 75) around the bars (1) is pooled through the holes (80, 80') and through the plate (85) and through holes (81) and screwed into the hole (81').

## Revendications

1. Le fixateur externe du squelette comprenant au moins deux broches (clous) (4) qu'on peut poser dans les deux fragments osseux principaux ; toute broche (4) du fixateur du squelette est fournie aussi de son propre support (2) qui tient le clamp (3), et le clamp (3) se pose sur un de ces broches (4), ce qui signifie que le clamp (3) se monte sur le support (2), permettant de cette manière le glissement du clamp (3) le long du support (2) et les mouvements rotatoires du clamp (3) autour du support (2), ce qui permet, d'autre part, l'ajustage du fixateur du squelette. Le support (2) est donc monté sur la barre de connexion (1), permettant l'ajustage du fixateur du squelette, et le glissement du support (2) le long de la barre de connexion (1) et les mouvements rotatoires du support (2) autour de la barre de connexion (1); la barre de connexion (1) relie au moins 2 supports (2).

2. Le fixateur externe du squelette conformément à l'exigence 1 du brevet est fourni de support (2) comprenant un corps circulaire ressemblant à une barre (8) avec la tête (9) cylindrique coaxiale intégrée de support sur son prolongement. La tête de support (9) est fournie de deux orifices bilatéraux (10 et 10') servant d'outils pour le montage de la barre de connexion (1). La vis (12), posée dans tête de support (9), comprend un élément de pression (13) qui se trouve à l'intérieur de la gouttière profilée (14) en forme de trou de serrure sur le corps de vis (12). L'élément de pression (13) comprend le cou cylindrique (15) avec la bille (16) sur une extrémité, et une surface concave sur l'autre ; le radius de la surface concave (18) et le radius de la barre de connexion (1) sont identiques; deux fentes (19, 19') disposées sur les surfaces opposées de l'élément de pression (13) portent les gouttières verticales (20, 20') qui se trouvent à l'intérieur de la tête cylindrique de support (9). La petite vis (21) ayant la tête ressemblant à la lentille, est posée sur l'extrémité libre de ce corps en forme de barre (8), ainsi que le diamètre de la tête, ressemblant à la lentille, devient supérieur par rapport au diamètre du corps ressemblant à la barre (8).

3. Le fixateur externe du squelette, conformément à l'exigence 2 du brevet, est fourni de vis (12) du support (2) qui comprend un élément de pression avec la surface concave et la bille (16A) qui porte une fente centrale (24). La bille (16A), par la pression, pénètre dans l'orifice plus petit (22) et se pose à l'intérieur de l'orifice sphérique (23) qui s'étend coaxialement avec le corps (12A) de la vis (12A).

4. Le fixateur externe du squelette, conformément aux exigences 1-3 du brevet, est fourni d'un support (2A) comprenant un tube circulaire (25) sur une extrémité et la tête cylindrique perpendiculaire de support (27) sur l'autre, avec un orifice (28) pour la vis (29) ayant une tête rectangulaire (30) et une surface concave (13A) qui entre dans l'orifice circulaire (28).

5. Le fixateur externe du squelette, conformément à l'exigence 4 du brevet, est fourni d'un support (2B) qui a la tête de support en forme de bille (31) sur une extrémité, avec un orifice cylindrique percé transversalement (32) par rapport à la barre de connexion (1). Donc, l'axe longitudinal de ce tube (25) et l'orifice cylindrique (32) sont en position perpendiculaire l'un par rapport à l'autre. La vis longue (29) avec l'élément de pression en forme du cylindre négatif (13A) pénètre et passe à travers le tube (25) en entrant dans l'orifice cylindrique (33).

6. Le fixateur externe du squelette, conformément aux exigences 1 ou 2 du brevet, est fourni d'un support (2C) comprenant une barre circulaire (34), et sur la barre (34) se trouve la tête de support en forme de parallélépipède (35) avec un orifice cylindrique percé transversalement (36) par rapport à la barre de connexion (1). La barre plus courte avec les pas (37) se trouve à l'extrémité opposée par rapport à la tête de support (35) ; la barre plus courte avec les pas est en position coaxiale avec l'axe du support (34) ; l'axe de la barre à filet (37) et la barre (34) sont en position perpendiculaire par rapport à l'axe de l'orifice cylindrique (36) de la tête de support (35).

7. Le fixateur externe du squelette, conformément à l'exigence 1 du brevet, est fourni de clamp (3) comprenant une vis spéciale (38) avec la tête cylindrique entaillée bilatéralement (39), et cette tête (39) comprend un orifice cylindrique (40) couvrant le corps (8) du support (2) par la barre de connexion (1). L'étal (41) entrant dans l'orifice cylindrique (40) par sa surface cylindrique négative (42) est posé sur l'extrémité entaillée de la tête cylindrique (39) de la vis. Derrière l'étal (41) se trouvent deux plaques circulaires annuaires (43) partiellement reliées qui couvrent la vis (38), et forment entre elles une gouttière (44) faite de cette manière pour permettre leur mise sur la broche (4). Les plaques (43) peuvent être séparées ou reliées faiblement. La plaque horizontale de l'étal (41) et la plaque (43) avec les surfaces dentelées (45 et 46) sont fixées par l'écrou (47) sur le corps de la vis à filet. (38)

8. Le fixateur externe du squelette, conformément à l'exigence 1, comprend la barre (5) composée d'un cylindre (48) et de la barre externe (49) coaxialement fixée, tandis que la gouttière (50) est posée sur le cylindre (48) de dessus ; donc, le cylindre (48) est partiellement télescopiquement introduit dans la barre de connexion (51) fixée par la vis de fixation (52) toute au long de la gouttière (50). Les barres de connexion (49,51) ont les mêmes diamètres que la barre de connexion (1), où la glissière (53) est fixée par une vis (54) et mise au-dessus du cylindre (48), tandis que l'autre glissière (55), fixée par une vis (56) est montée sur la barre externe (51). Les prolongements (57, 57') de ces glissières sont fixés par un fuseau à filet (58) et la tête de la vis (59) est fixée au milieu du fuseau à filet (58). L'échelle de mesure est gravée entre les glissières (53,55) sur la barre de connexion (51). Sur la barre de connexion (49) se trouve une paire de supports (2), retirés par les clamps (3) et attachés à des broches (4) dans un fragment de l'os, tandis que l'autre paire de supports (2) avec des clamps fixés aux broches (4) se trouve dans l'autre fragment principal de l'os.

9. Le fixateur externe du squelette, conformément à l'exigence 8, est fourni d'un dispositif en forme de plaque (6) composé d'une plaque supérieure (62) avec le fuseau horizontal rotatif à filet (63) et la tête hexagonale (64), et d'une plaque inférieure (68) avec les orifices demi-cylindriques (65, 69) qui entourent la barre de connexion (1) et se trouvent sur ces plaques (62,68). Les plaques (62,68) sont bien fixées par des vis cylindriques (67,67') entrant dans les orifices cylindriques (66,66') sur la plaque supérieure (62) et dans les orifices à filet (70,70') sur la plaque inférieure (68). Le fuseau à filet (63) est fixé sur la plaque (71) par le serrage, à travers l'orifice à filet (72) dont les gouttières transversalement concaves (73,73') sont réciproques et s'utilisent pour appuyer la tête (9) du support (2).

10. Le fixateur externe du squelette, conformément à l'exigence 1, comprend un clamp double (7) avec deux clamps (74,75) et les orifices cylindriques (76,77) pour les barres de connexion (1). Les orifices (76 ,77) sont coupés à un endroit et prolongés sur les deux extrémités (78,78') du clamp (74), et les extrémités (79,79') de l'autre clamp (75) portent les orifices circulaires (80,80') percés coaxialement par les deux extrémités (78, 78'), tandis que les autres orifices circulaires (81, 81') sont percés par d'autres extrémités (78', 79) qui se trouvent sur les côtés opposés. Les deux orifices ont les dents radialement disposées (83,84), tandis que la plaque (85) dentelée de deux côtés, est introduite entre deux orifices circulaires (81'84). La vis (82) pour la fixation des clamps (74,75) autour des barres (1) est glissée à travers les orifices (80,80'), à travers la plaque (85) et les orifices (81) et vissé dans l'orifice (81').

## Patentansprüche

1. Ein externer Knochen-Fixierer, der mindestens zwei Pins (4) aufweist, die in jedem Hauptknochenfragment befestigbar sind, wobei der Knochenfixierer weiters für jeden Pin (4) einen Träger (2) aufweist, der eine Klemme (3) trägt, die den Pin (4) hält, wobei die Klemme (3) an dem Träger (2) derart montiert ist, dass zum Zweck der Justierung des Knochenfixierers eine Gleitbewegung der Klemme (3) entlang dem Träger (2) und eine Drehbewegung der Klemme (3) um den Träger (2) ermöglicht ist, und wobei der Träger (2) an einem Verbindungsstab (1) so befestigt ist, dass zum Zweck der Justierung des Knochenfixierers eine Gleitbewegung des Trägers (2) entlang dem Verbindungsstab (1) und eine Drehbewegung des Trägers (2) um den Verbindungsstab (1) ermöglicht ist, wobei der Verbindungsstab (1) zumindest zwei Träger (2) miteinander verbindet.

2. Ein externer Knochen-Fixierer nach Anspruch 1 **dadurch gekennzeichnet, dass** der Träger (2) einen runden stabförmigen Körper (8) mit koaxial und integral verlaufendem zylindrischem Tragkopf (9) aufweist, wobei der Tragkopf (9) zweiseitige Löcher (10, 10') zur Aufnahme für den Verbindungsstab (1) aufweist, wobei eine Schraube (12) in den Tragkopf (9) eingedreht ist, wobei die Schraube ein Druck-Element (13) aufweist, welches einer profilierten, schlüssellochähnlichen Nut (14) an einem Schaft der Schraube (12) platziert ist, wobei das Druck-Element (13) einen zylinderförmigen Hals (15) aufweist, der an einem seiner Enden eine Kugel (16) und an seinem anderen Ende eine konkave Oberfläche aufweist, wobei der Durchmesser der konkaven Oberfläche (18) und der des Verbindungsstabs (1) identisch sind, wobei zwei Nasen (19, 19') auf einander gegenüberliegenden Oberflächenbereichen des Druck-Elements (13) zur Abstützung vertikaler Nuten (20, 20') im Inneren des zylinderförmigen Tragkopfes (9) vorgesehen sind, und wobei an einem freien Ende des stabförmigen Körpers (8) eine kleine Schraube (21) mit linsenförmigem Kopf eingedreht ist, wobei der Durchmesser des linsenförmigen Kopfes größer als der des stabförmigen Körpers (8) ist

3. Ein externer Knochen-Fixierer nach Anspruch 2, **dadurch gekennzeichnet, dass** die Schraube (12A) des Trägers (2) ein Druck-Element (13A) mit einer konkaven Oberfläche und einer Kugel (16A) aufweist, wobei die Kugel einen Schlitz (24) aufweist, wobei die Kugel (16A), die durch eine schmalere Öffnung (22) gedrückt ist, in einem sphärischen Loch (23) platziert ist, das sich koaxial zu dem Hals der Schraube (12A) erstreckt.

4. Ein externer Knochen-Fixierer nach einem der Ansprüche 1 bis 3, **dadurch gekennzeichnet, dass** der Träger (2A) einen runden, röhrenförmigen Körper (25) auf einem Ende und einen zylindrischen Tragkopf (27) mit einer kreisrunden Öffnung (28) für eine Schraube (29) mit einer konkaven Fläche, welche durch ein kreisförmiges Loch (28) gesteckt ist, befestigt, wobei die Schraube (29) eine rechtwinklige Form aufweist.

5. Ein externer Knochen-Fixierer nach Anspruch 4, **dadurch gekennzeichnet, dass** der Träger (2B) an einem seiner Enden einen kugelförmigen Tragkopf (31) mit einer transversal durchgebohrten, zylindrischer Öffnung (32) für den Verbindungsstab (1), wobei die in der Längsrichtung verlaufenden Symmetrieachsen des röhrenförmigen Körpers (25) und der Öffnung (32) aufeinander senkrecht stehen, wobei in den röhrenförmigen Körper (25) eine lange Schraube (29) mit einem Druck-Element, welches die Form eines negativen Zylinders (13) aufweist und das zylindrische Loch penetriert, durch den röhrenförmigen Körper geschoben und eingedreht ist.

6. Ein externer Knochen-Fixierer nach einem der Ansprüche 1 oder 2, **dadurch gekennzeichnet, dass** der Träger (2C) einen runden Stab (34) aufweist, auf welchem Stab (34) ein Tragkopf (35) in Form eines Parallelepipeds mit einer quer durchgebohrten zylindrischen Öffnung (36) für den Verbindungsstab (1) angeordnet ist, wobei an dem entgegengesetzten Ende des Tragkopfs (35), koaxial mit dem stabförmigen Körper (34), ein kürzerer Stab mit Gewinden (37) angeordnet ist, wobei die Achsen des Stabes mit Gewinde (37) und des Trägers (34) senkrecht auf der Längsachse der zylindrischen Öffnung (36) des Tragkopfes (35) stehen.

7. Ein externer Knochen-Fixierer nach Anspruch 1 **dadurch gekennzeichnet, dass** die Klemme (3) des Knochen-Fixierer aus einer speziellen Schraube (38) mit beidseitig gekerbtem walzenförmigem Kopf (39) besteht, und dieser Kopf mit einer quer durchgebohrten zylindrischen Öffnung (40) für eine Anbringung an dem Körper (8) des Trägers oder am Verbindungstab (1) versehen ist, wobei ein Reiter (41), der mit seiner kalottenförmigen Fläche (42) in die zylindrische Öffnung (40) hineingeht, über einen gekerbten Teil des walzenförmigen Kopfes (39) der Schraube (38) gestreift ist, wobei nach dem Reiter (41) zwei teilweise verbundene kreisrund-ringförmige Platten (43) an der Schraube (38) angebracht sind, diese Platten haben auf der Verbindungsstelle eine geformte Nut (44) zur Anbringung an einem Pin (4), wobei die Platten (43) getrennt oder lose verbunden sein können, wobei die Auflagefläche des Reiters (41) und eine Platte (43) mit gezähnter Oberfläche (45, 46) durch die auf den Bolzen mit Gewinde (38) geschraubte Mutter (47) fest angezogen ist.

8. Ein externer Knochen-Fixierer nach Anspruch 1, **dadurch gekennzeichnet, dass** er einen Träger (5) aufweist, der sich aus einem Zylinder (48) mit einem koaxial befestigten externen Stab (49) zusammensetzt, wobei sich eine längere Nut (50) oben auf dem Zylinder (48) befindet, wobei der Verbindungsstab (51) teilweise in den Zylinder (48) teleskopartig eingesetzt ist, wobei der Verbindungsstab (51) mit Hilfe von einer Schraube (52) durch die Nut (50) fest angezogen wird, wobei die Verbindungsstäbe (49, 51) den gleichen Durchmesser wie der Verbindungsstab (1) aufweisen, wobei eine Gleitvorrichtung (53) an dem Zylinder (48) mittels einer Schraube (54) befestigt ist, wobei an dem externen Verbindungsstab (51) eine mittels einer Schraube befestigte eine weitere Gleitvorrichtung (53) angeordnet ist, wobei Fortsätze (57, 57') der Gleitvorrichtungen (53, 55) mittels einer Gewindespindel (58) fixiert sind, wobei ein Kopf einer Schraube (59) in der Mitte der Gewindespindel (58) befstigt ist, und wobei eine Skala in der zwischen den Gleitvorrichtungen (53, 55) auf der Verbindungsstange (51) angeordnet ist, und dass ein Paar von Trägern (2) mit Klemmen (3), welche mit den Pins (4) eines Knochenfragmentes verbunden sind, auf die Verbindungsstange (49) geschoben ist, wohingegen ein andere Paar von Trägern (2) mit seinen Klemmen mit den Pins (4) eines anderen Hauptknochenfragmentes verbunden ist.

9. Ein externer Knochen-Fixierer nach Anspruch 8, **dadurch gekennzeichnet, dass** er eine plattenförmigen Vorrichtung (6), die sich aus einer Oberplatte (62) mit drehbar gelagerter Schraubenspindel (63) mit sechskantigem Kopf (64) und einer Unterplatte (68) zusammensetzt, die halbzylindrische Öffnungen (65, 69) aufweist, zur Umschließung des Verbindungsstab (1), wobei die Platten (62, 68) mit den Schrauben (67, 67') durch die zylindrischen, auf der Oberplatte (62) befindlichen Öffnungen (66, 66') und durch die auf der Unterplatte (68) befindlichen Öffnungen mit Gewinden (70, 70') befestigt sind, wobei die Schraubenspindel (63) in die profilierte Platte (71) durch die Öffnung mit Gewinden (72) eingeschraubt ist, deren konkave Nuten (73, 73'), zum Stützen des runden Tragkopfes (9) des Trägers (2) dienen.

10. Ein externer Knochen-Fixierer nach Anspruch 1, **dadurch gekennzeichnet, dass** er eine Doppelklemme (7) aufweist, die aus zwei Klemmen (74, 75) mit zylindrischen Öffnungen (76, 77) für Verbindungsstäbe (1) besteht, wobei die Öffnungen (76, 77) an einer Stelle so geschnitten sind, dass sich die Klemme (74) in zwei Schenkel (78, 78') und die Klemme (75) in zwei andere Schenkel (79, 79') teilt, durch die beiden Schenkel (78, 78') sind koaxial die kreisförmigen Öffnungen (80, 80') durchgebohrt, und durch die Schenkel (79, 79') sind auch die kreisförmigen Öffnungen (81, 81') durchgebohrt, wobei die runde Öffnung (81) mit Gewinde versehen ist; die Öffnungen (80', 81) durch die einander gegenüberliegenden Schenkel (78', 79) sind mit radial und strahlenförmig verlaufenden Zähnen (83, 84) versehen und zwischen ihnen wird ein beidseitig radial strahlenförmig gezähntes, rund-ringförmiges Plättchen (85) eingesetzt, die Schraube (82) zum Anziehen der Klemmen (74, 75) um die Verbindungsstäbe (1) ist durch die Öffnungen (80, 80'), durch das Plättchen (85) und durch die Öffnungen (81, 81') durchgezogen.
